# EUROPÄISCHE PATENTSCHRIFT

(11) **EP 1 496 993 B1**
(45) Veröffentlichungstag und Bekanntmachung des Hinweises auf die Patenterteilung: **11.08.2010**
(21) Anmeldenummer: 03727209.3
(22) Anmeldetag: 23.04.2003
(51) Int. Cl.: A61N 5/06

(54) **LICHTAPPLIKATOR UND VERFAHREN ZUR HERSTELLUNG EINES STREUKÖRPERS**
LIGHT APPLICATOR AND METHOD FOR PRODUCING A DIFFUSER
APPLICATEUR DE LUMIERE ET PROCEDE DE FABRICATION D'UN DIFFUSEUR

(30) Priorität: 24.04.2002 DE 20206473 U; 29.11.2002 DE 10256139
(43) Veröffentlichungstag der Anmeldung: 19.01.2005
(73) Patentinhaber: Ludwig-Maximilians-Universität, 80539 München (DE)
(72) Erfinder: BEYER, Wolfgang, 82166 Gräfelfing (DE); OBERMEIER, Andreas, 80939 München (DE)
(74) Vertreter: Herrmann, Franz
(86) Internationale Anmeldenummer: PCT/DE2003/001318
(87) Internationale Veröffentlichungsnummer: WO 2003/090866

(56) Entgegenhaltungen:
- EP-A- 0 437 183
- EP-A- 0 439 629
- DE-A- 3 901 931
- US-A- 5 269 777
- US-A- 5 957 917

## Beschreibung

Die Erfindung betrifft einen Lichtapplikator mit einem an einen Lichtleiter anbringbaren Streukörper, bei dem verschiedene Streubereiche mit unterschiedlichen Streuparametern entlang der in den Streukörper hinein verlängerten optischen Achse des Lichtleiters aufeinander folgen, wobei die Streubereiche bezüglich einer im rechten Winkel zur optischen Achse des Lichtleiters ausgerichteten Sichtlinie überlappen.

Die Erfindung betrifft ferner ein Verfahren zur Herstellung eines an einen Lichtleiter anschließbaren Streukörpers.

Aus der EP 0 439 629 A1 sind ein derartiger Lichtapplikator und ein derartiges Verfahren bekannt. Der bekannte Lichtapplikator verfügt über einen Sondenkörper, der aus einem synthetischen Material gefertigt ist, in das Streupartikel eingebracht sind. In den Probenkörper ist ferner ein zugespitztes Ende einer Lichtleitfaser eingebracht, das mit einer äußeren Streuschicht versehen ist.

Ferner sind aus der US 5 978 541 ein weiterer Lichtapplikator für medizinische Anwendungen und ein weiteres Verfahren zur Herstellung eines an einen Lichtleiter anschließbaren Streukörpers bekannt. Der bekannte Lichtapplikator weist einen zylindrischen Kern auf, der mit Streupartikeln durchsetzt ist. Die Streupartikel dienen als Streuzentren, an denen das durch den Lichtleiter in den Streukörper einfallende Licht gestreut wird.

Die Konzentrationsverteilung der Streuzentren entlang der in den Streukörper hinein verlängerten optischen Achse des an den Streukörper anschließbaren Lichtleiters ist so gewählt, dass der Streukörper mit einer vorbestimmten Lichtverteilung leuchtet.

Der Streukörper wird in einem Extrusionsvorgang hergestellt, bei dem die Konzentration der Streuzentren durch Mischen zweier Suspensionen mit unterschiedlichen Konzentrationen eingestellt wird. Zur Herstellung eines bestimmten Konzentrationsverlaufs wird die Konzentration der Streuzentren während des Extrusionsvorgangs kontinuierlich überwacht und mit einem vorgegebenen Sollwert verglichen.

Zur Ermittlung der Sollwerte wird vorgeschlagen, Prototypen des Streukörpers aus einzelnen Teilen mit unterschiedlichen Konzentrationen zusammenzusetzen. Aus der Vielzahl der Prototypen kann dann derjenige Prototyp ausgesucht werden, dessen Lichtverteilung der gewünschten Lichtverteilung am ehesten entspricht. Der Mischvorgang während der Extrusion des Streukörpers wird dann so eingestellt, dass der fertige Streuköper näherungsweise die ausgewählte Verteilung der Streuzentren aufweist.

Die bekannten Lichtapplikatoren werden allgemein im Rahmen der photodynamische Therapie zur Behandlung von Tumoren verwendet. Dabei wird ein Photosensibilisator, der sich selektiv im Tumor anreichert, appliziert. Nach der Applikation des Photosensibilisators wird der Tumor und das umgebende gesunde Gewebe mit Licht bestrahlt. Durch die dadurch ausgelösten photochemischen Prozesse werden Toxine erzeugt, die aufgrund der Tumorselektivität gezielt den Tumor schädigen.

Da sich auch im gesunden Gewebe eine gewisse, wenn auch geringe Konzentration des Photosensibilisators einstellt, kann eine Überdosierung mit Licht zu unerwünschten Gewebeschäden im gesunden Gewebe führen. Andererseits bleibt bei einer Unterdosierung der gewünschte Therapieerfolg aus. Der Toleranzbereich für die zu applizierende Lichtdosis ist daher oft schmal. Da die Lichtverteilung von der Verteilung der Streuzentren im Streukörper abhängt, ist für eine bestimmte Lichtverteilung eine bestimmte Konzentrationsverteilung der Streuzentren im Streukörper erforderlich. Damit die notwendige Genauigkeit bei der Konzentration der Streuzentren erreicht werden kann, benötigt das bekannte Verfahren zur Herstellung des Streukörpers eine aufwändige Regelung für den Extrusionsvorgang.

Ausgehend von diesem Stand der Technik liegt der Erfindung daher die Aufgabe zugrunde, einen einfach herstellbaren

Lichtapplikator zu schaffen und ein Verfahren zur Herstellung eines für den Lichtapplikator verwendbaren Streukörpers mit einer definierten Konzentrationsverteilung der Streuzentren anzugeben.

Diese Aufgaben werden durch den Lichtapplikator und das Verfahren mit den Merkmalen der unabhängigen Ansprüche gelöst. In davon abhängigen Ansprüchen sind weitere Ausgestaltungen und Weiterbildungen angegeben.

Bei dem Lichtapplikator ist der Streukörper derart ausgebildet, dass die Streubereiche bezüglich einer im rechten Winkel zur optischen Achse des Lichtleiters ausgerichteten Sichtlinie überlappen. Eine Querschnittsfläche, deren Normale die optische Achse ist, setzt sich daher im Überlappungsbereich der Streubereiche aus Teilflächen mit unterschiedlichen Streuparametern zusammen. Das entlang der optischen Achse einfallende Licht wird daher auf unterschiedliche Teilflächen mit unterschiedlichen Streuparametern treffen. Das Flächenverhältnis der Streubereiche in der jeweiligen Querschnittsfläche kann entsprechend der gewünschten Lichtintensität gewählt werden. Bei dem Lichtapplikator findet somit nicht ein Mischvorgang der verschiedenen Streumedien bei der Herstellung, sondern eine Mischung der in verschiedenen Streubereichen gestreuten Lichtanteile statt.

Da die unterschiedliche Streuparameter aufweisenden Streubereiche räumlich getrennt sind, können die Streuparameter der einzelnen Streubereiche bei der Herstellung jeweils separat mit großer Genauigkeit auf die geforderten Werte eingestellt werden. Es ist zur Herstellung des Streukörpers für den Lichtapplikator insbesondere nicht notwendig, einen komplexen Mischvorgang durchzuführen und zu überwachen. Vielmehr können die Streumedien für die verschiedenen Streubereiche mit den unterschiedlichen Streuparametern separat hergestellt und zu dem gemeinsamen Streukörper verbunden werden. Der Lichtapplikator lässt sich daher auf einfache Weise so herstellen, dass ein vorbestimmtes Abstrahlprofil eingehalten wird.

Bei einer bevorzugten Ausführungsform des Lichtapplikators sind die Grenzflächen paraboloidförmig ausgebildet, wobei sich die Symmetrieachsen der Paraboloide entlang der in das Streumedium verlängerten optischen Achse des Lichtleiters erstrecken. Da sich die Querschnittsfläche der Paraboloide linear mit der entlang der optischen Achse zurückgelegten Wegstrecke ändert, kann mit einer derartigen Anordnung ein linearer Übergang zwischen zwei Streubereichen mit unterschiedlichen Streuparametern bewerkstelligt werden. Außerdem können die Streubereiche durch Injizieren eines ersten Streumediums in ein zweites Streumedium hergestellt werden, wobei unter Injizieren sowohl Einsaugen als auch Einspritzen verstanden werden soll.

In einer weiteren bevorzugten Ausführungsform ist einem proximalen Ende des Streukörpers ein Reflektor zugeordnet, der das vom Streukörper ausgehende Licht bevorzugt in vorgegebene Richtungen lenkt. Ein derartiger Reflektor kann beispielsweise eine streuende Halbkugel sein, die das vom Streukörper ausgehende Licht zum distalen Ende hin lenkt. Ein derartiger Lichtapplikator eignet sich insbesondere im Rahmen der Gynäkologie zur photodynamischen Therapie von Dysplasien auf der Oberfläche von Portio und Zervix-Kanal.

Nachfolgend wird die Erfindung im Einzelnen anhand der beigefügten Zeichnung erläutert. Es zeigen:
- Figur 1: einen Querschnitt durch ein erstes Aus- führungsbeispiel eines Streukörpers und ein Diagramm mit der über den Querschnitt gemittelten Konzentration der Streuzent- ren im Streukörper;
- Figur 2: einen Querschnitt durch einen weiteren abgewandelten Streukörper;
- Figur 3: einen Querschnitt durch einen Streukör- per, dessen distales Ende mit dem Spiegel abgeschlossen ist;
- Figur 4a bis 4e: Darstellungen der zur Herstellung des Streukörpers aus Figur 2 angewandten Ver- fahrensschritte;
- Figur 5: einen Querschnitt durch einen Lichtappli- kator für Portio und Zervix-Kanal;
- Figur 6: einen Querschnitt durch einen weiteren abgewandelten Lichtapplikator; und
- Figur 7: einen Querschnitt durch einen Lichtappli- kator, dessen Lichtaustrittsfläche mit einer partiell rückstreuenden Schicht versehen ist.

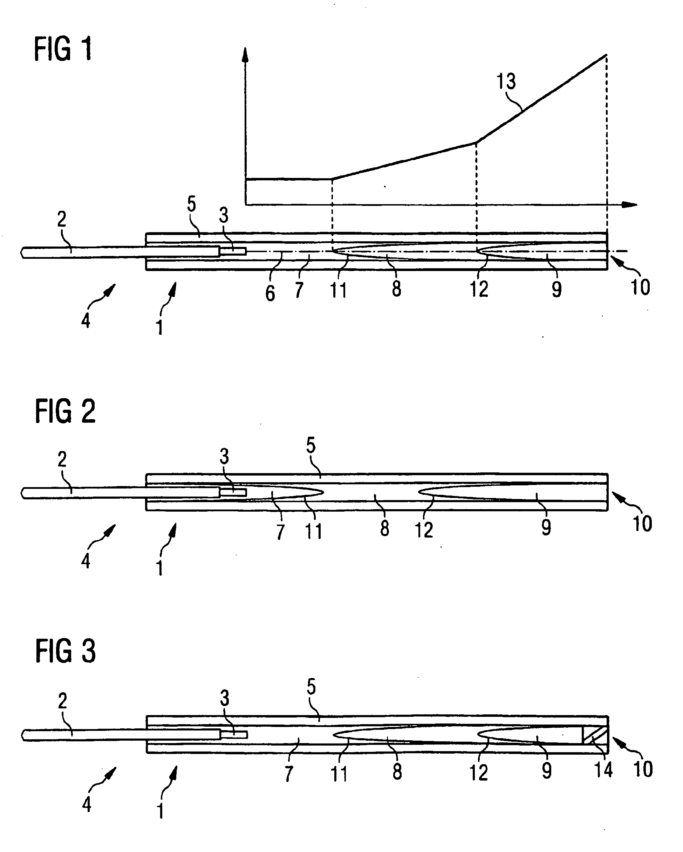

Der in Figur 1 dargestellte Streukörper 1 ist an einen Lichtleiter 2 anschließbar. Der Lichtleiter 2 ragt dabei mit einer Lichtleitfaser 3 in das proximale Ende 4 eines Schlauchstücks 5 hinein. In Figur 1 ist eine optische Achse 6 der Lichtleitfaser 3 in das Innere des Schlauchstücks 5 hinein verlängert gezeichnet.

Entlang der in das Innere des Schlauchstücks 5 hinein verlängerten optischen Achse 6 sind nacheinander ein proximaler Streubereich 7, ein mittlerer Streubereich 8 und ein distaler Streubereich 9 ausgebildet. Der distale Streubereich 9 schließt ein distales Ende 10 des Schlauchstücks 5 ab. Der proximale Streubereich 7, der mittlere Streubereich 8 und der distale Streubereich 9 sind jeweils durch paraboloidförmige Grenzflächen 11 und 12 voneinander abgegrenzt. Bei dem in Figur 1 dargestellten Ausführungsbeispiel des Streukörpers 1 sind die Paraboloidscheitel der paraboloidförmigen Grenzflächen 11 und 12 jeweils dem proximalen Ende 4 des Streukörpers 1 zugewandt. Ferner liegen die Symmetrieachsen der paraboloidförmigen Grenzflächen 11 und 12 auf der optischen Achse 6. Da die im rechten Winkel zur optischen Achse 6 ausgerichteten Querschnittsflächen der paraboloidförmigen Grenzflächen 11 und 12 proportional zum Abstand vom Paraboloidscheitel sind, nimmt die flächenmäßig über die Querschnittsfläche gemittelte Konzentration der Streuzentren linear mit dem Abstand vom Paraboloidscheitel der paraboloidförmigen Grenzflächen 11 und 12 je nach Konzentration der Streuzentren in dem proximalen Streubereich 7, dem mittleren Streubereich 8 und dem distalen Streubereich 9 zu oder ab.

Die Konzentration der Streuzentren im proximalen Streubereich 7, im mittleren Streubereich 8 und im distalen Streubereich 9 werden nachfolgend mit c₁, c₂ und c₃ bezeichnet. Die Konzentration der Streuzentren im proximalen Streubereich 7, im mittleren Streubereich 8 und im distalen Streubereich 9 können stark unterschiedliche Werte annehmen.

Durch den linearen Übergang der flächenmäßigen Anteile der unterschiedlichen Streubereiche 7, 8 und 9 ergibt sich ein Konzentrationsverlauf 13, wie er beispielhaft in dem in Figur 1 dargestellten Diagramm gezeigt ist. In dem in Figur 1 dargestellten Diagramm ist entlang der Ordinate der Weg entlang der optischen Achse 6 aufgetragen. Die Abszisse zeigt die über die Querschnittsfläche gemittelte Konzentration der Streubereiche 7 bis 9.

Wenn die Konzentration der Streuzentren vom proximalen Streubereich 7 zum distalen Streubereich 9 hin ansteigt, also c₁ < c₂ < c₃ gilt, ergibt sich der in Figur 1 dargestellte, abschnittsweise lineare, stetig ansteigende Konzentrationsverlauf 13 der Streuzentren. Die Streuwahrscheinlichkeit nimmt daher von proximalen Ende 4 zum distalen Ende 10 hin zu. Mit Hilfe des Konzentrationsverlaufs 13 lässt sich die vom proximalen Ende 4 zum distalen Ende 10 hin abnehmende Intensität des entlang der optischen Achse 6 verlaufenden Lichts ausgleichen. Im Ergebnis wird daher die Intensität des aus dem Streukörper 1 herausgestreuten Lichts vom proximalen Ende 4 zum distalen Ende 10 kaum abnehmen. Es lässt sich daher eine homogene Lichtverteilung entlang der optischen Achse 6 des Streukörpers 1 erzielen. Unter einer homogenen Lichtverteilung soll vorzugsweise eine Lichtverteilung verstanden werden, bei der die Leistungsdichte auf den Licht emittierenden Oberflächen des Streukörpers 1 um maximal +/-15 %, vorzugsweise +/-10 % schwankt.

In Figur 1 ist der Paraboloidscheitel der paraboloidförmigen Grenzflächen 11 und 12 jeweils dem proximalen Ende 4 des Streukörpers 1 zugewandt. Dies ist jedoch nicht unbedingt erforderlich. Figur 2 zeigt ein abgewandeltes Ausführungsbeispiel des Streukörpers 1, bei dem die Grenzfläche 11 zwischen dem proximalen Streubereich 7 und dem mittleren Streubereich 8 ein Paraboloid ist, dessen Paraboloidscheitel zum distalen Ende 10 weist. Mit dieser Anordnung der Streubereiche 7 bis 9 lassen sich Konzentrationsverläufe herstellen, die mit der gleichgerichteten Orientierung der Streubereiche 7 bis 9 nicht herstellbar sind. Zum Beispiel kann der mittlere Streubereich 8 des in Figur 2 dargestellten Streukörpers 1 frei von Streuzentren sein, so dass sich im Konzentrationsverlauf der Streuzentren ein ausgeprägtes Minimum ergibt.

Figur 3 zeigt ein weiteres abgewandeltes Ausführungsbeispiel des Streukörpers 1, bei dem am distalen Ende 10 des Streukörpers 1 ein Spiegel 14 in das Schlauchstück 5 eingebracht ist. Durch den Spiegel 14 wird ein Austreten von Licht aus dem distalen Ende des Streukörpers 1 verhindert.

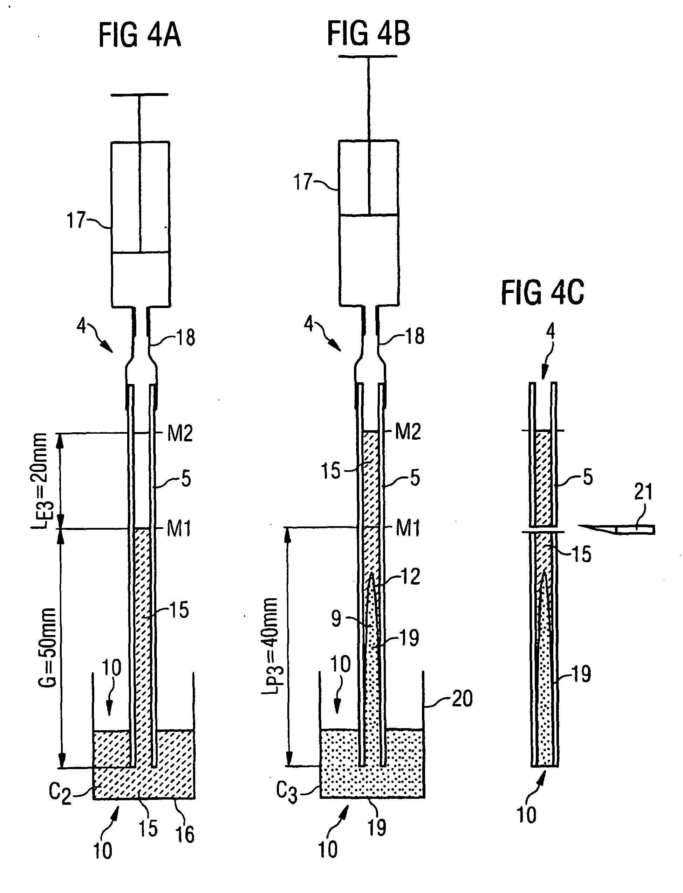

In den Figuren 4a bis 4e sind aufeinanderfolgende Verfahrensschritte zur Herstellung des in Figur 2 dargestellten Streukörpers 1 dargestellt.

Die Streubereiche 7 bis 9 im Schlauchstück 5 werden allgemein dadurch ausgebildet, dass ein aushärtbares, flüssiges Streumedium, dem Streupartikel beigemischt sind, in das Schlauchstück 5 eingebracht wird.

Vor dem Befüllen des Schlauchstücks 5 werden an dem Schlauchstück 5 Markierungen M1 und M2 angebracht. Die Markierungen M1 und M2 sind in einem Abstand L_{E3} = 20 mm angeordnet. Da die Querschnittsfläche eines Paraboloids proportional zum Abstand vom Paraboloidscheitel anwächst, ist das Volumen eines Paraboloids gleich der Querschnittsfläche mal dem halben Abstand vom Paraboloidscheitel oder anders ausgedrückt gleich der Grundfläche mal der halben Höhe des Paraboloids. Das mit den Markierungen M1 und M2 markierte Volumen im Schlauchstück 2 entspricht dem Volumen eines in das Schlauchstück 5 einzusaugenden Streumediums mit der Höhe L_{P3} = 40 mm.

Zunächst wird aber ein erstes für den mittleren Streubereich 8 vorgesehenes Streumedium 15 aus einem Behälter 16 bis zu der Markierung M1 in das Schlauchstück 5 eingesaugt. Zum Einsaugen des Streumediums 15 in das Schlauchstück 5 wird eine Saugpumpe 17 verwendet, die über ein Schlauchstück 18 mit dem proximalen Ende 4 des Schlauchstücks 5 verbunden ist.

Vor dem Aushärten des Mediums 15 wird nunmehr gemäß Figur 4b ein weiteres, für den distalen Streubereich 9 vorgesehenes Streumedium 19 aus einem Behälter 20 in das Schlauchstück 5 eingesaugt. Aufgrund der laminaren Strömung des Streumediums 19 dringt das Streumedium 19 im mittleren Bereich des Schlauchstücks weiter in das Streumedium 15 vor als am Rand. Es entsteht daher die paraboloidförmige Grenzfläche 12 zwischen dem distalen Streubereich 9 und dem mittleren Streubereich 8. Beim Einsaugen des Streumediums 19 in das Schlauchstück 5 wird der Flüssigkeitsspiegel des Streumediums 15 von der Markierung M1 bis zur Markierung 2 angehoben. Dadurch ergibt sich ein distaler Streubereich 9, dessen Volumen dem zwischen den Markierungen M1 und M2 liegenden Volumen des Schlauchstücks 5 entspricht. Die Länge L_{P3} des distalen Streubereichs 9 beträgt daher das Doppelte des Abstands L_{E3} zwischen Markierungen M1 und M2. Durch Einstrahlen von Licht in die Streumedien 15 und 19 können die in das Schlauchstück 5 eingesaugten Streumedien 15 und 19 auf Blasenfreiheit und die Grenzfläche 12 auf eine fehlerfreie Ausbildung überprüft werden.

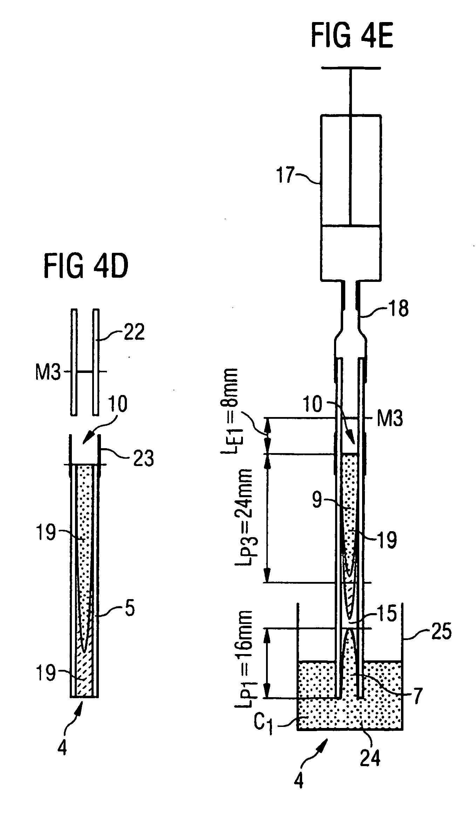

Anschließend wird gemäß Figur 4c das Schlauchstück 5 auf die Gesamtlänge G = 50 mm mit Hilfe einer Schneidevorrichtung 21 zugeschnitten. In einem weiteren in Figur 4d gezeigten Verfahrensschritt wird das Schlauchstück 5 um 180° gedreht und an das distale Ende 10 ein Verlängerungsstück 22 mit Hilfe eines Verbindungsstücks 23 angebracht. Das Verlängerungsstück 22 ist mit einer Markierung M3 versehen, die sich in einer Entfernung L_{E1} = 8 mm vom Ende des Schlauchstücks 5 befindet. Die Markierung M3 markiert die Einsauglänge für das Ausbilden des proximalen Streubereichs 7. Diese wird erzeugt, indem gemäß Figur 4e vom proximalen Ende 4 des Schlauchstücks 5 her ein für den proximalen Streubereich 7 vorgesehene Streumedium 24 aus einem Behälter 25 eingesaugt wird. Der paraboloidförmige distale Streubereich 9 bewegt sich dabei bis zur Markierung M3 zurück.

In weiteren nicht dargestellten Verfahrensschritten werden das distale Ende 10 beispielsweise durch den Spiegel 14 verschlossen und der Lichtleiter 2 in das proximale Ende 4 des Streukörpers 1 der eingeführt und die Streumedien 15, 19 und 24 ausgehärtet. Dabei wird die Lichtleitfaser 3 im proximalen Streubereich 7 fixiert.

Die anhand der Figuren 1 bis 3 dargestellten Streukörper 1 können bereits in der vorliegenden Form als Lichtapplikatoren zum Bestrahlen von Hohlorganen verwendet werden. Daneben können die Streukörper 1 für spezielle Anwendungen weiter modifiziert werden.

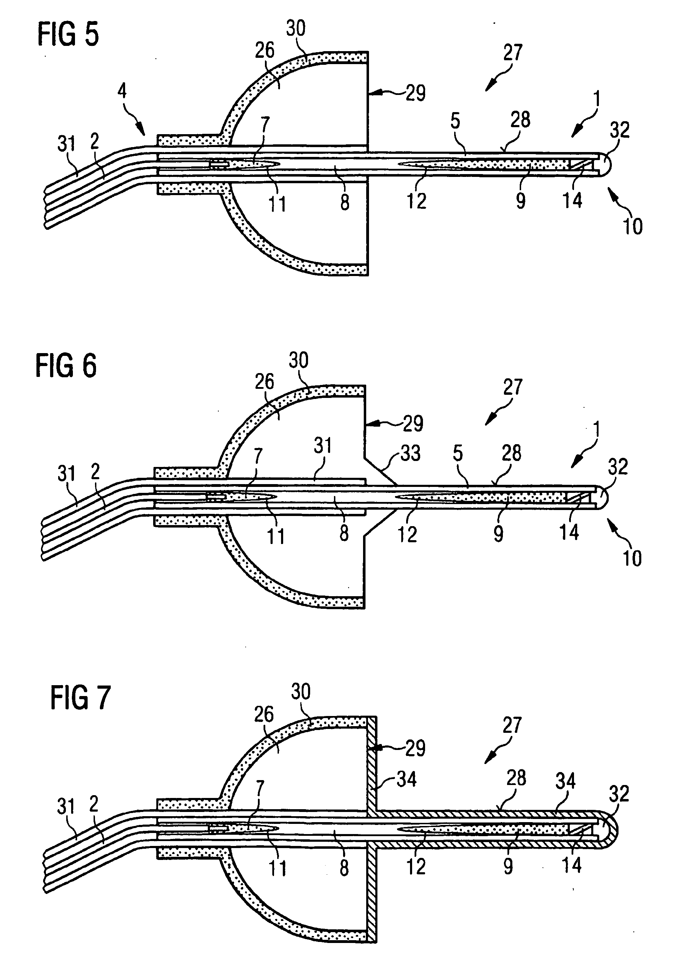

Die Streukörper 1 lassen sich zum Beispiel im Rahmen der Gynäkologie als Lichtquellen zum Bestrahlen von Portio und Zervix-Kanal verwenden. Zu diesem Zweck wird zum Beispiel der Streukörper 1 aus Figur 2, wie in Figur 5 dargestellt, mit einer transparenten Halbkugel 26 kombiniert, die am proximalen Ende 4 des Streukörpers 1 angeordnet ist. Der Streukörper 1 bildet zusammen mit der Halbkugel 26 einen Lichtapplikator 27, dessen Lichtaustrittsfläche von einer Schlauchoberfläche 28 des Schlauchstücks 5 und einer Halbkugelquerschnittsfläche 29 der Halbkugel 26 gebildet ist. Um ein unerwünschtes Bestrahlen der Scheidenwand zu verhindern, ist die rückseitige Oberfläche der Halbkugel 26 mit einer reflektierenden oder vollständig zurückstreuenden Reflexionsschicht 30 versehen.

Die Halbkugel 26 sitzt nicht unmittelbar auf dem Streukörper 1 auf. Vielmehr ist der Streukörper 1 in ein durchsichtiges Rohr 31 eingebettet, das aus Gründen der Anatomie unmittelbar hinter der Halbkugel 26 um etwa 30° abgewinkelt ist. An dem Rohr 31 ist auch ein in Figur 5 nicht dargestellter Handgriff angebracht, mit dessen Hilfe der Arzt den Lichtapplikator 27 manipulieren kann. Um schließlich die Verletzungsgefahr zu eliminieren, ist das distale Ende 10 des Streukörpers 1 mit einer runden Kappe 32 abgeschlossen.

Bei dem in Figur 5 dargestellten Ausführungsbeispiel ist die Konzentration der Streuzentren im proximalen Streubereich 7 und im distalen Streubereich 9 größer als im mittleren Streubereich 8. Die Konzentration der Streuzentren nimmt daher vom proximalen Ende 4 her zum mittleren Streubereich 8 hin ab und steigt zum distalen Ende 10 hin erneut an. Durch diese Wahl der Konzentrationsverhältnisse wird eine starke Abstrahlung aus dem Streukörper 1 im Bereich der Halbkugel 26 erzielt. Die Halbkugel 26 wird gewissermaßen durch den proximalen Streubereich 7 mit Licht versorgt. Der distale Streubereich 9 sorgt dagegen dafür, dass aus der Schlauchoberfläche 28 eine ausreichende Menge an Licht austritt. Da die über den Querschnitt des Streukörpers 1 gemittelte Konzentration der Streuzentren zum distalen Ende 10 des Streukörpers 1 hin zunimmt, wird der Abfall des entlang der optischen Achse 6 einfallenden Lichts kompensiert. Durch eine geeignete Wahl der Konzentration im distalen Streubereich 9 und im mittleren Streubereich 8 lässt sich eine homogene Lichtverteilung entlang über den Streukörper 1 erzielen. Die homogene Lichtverteilung über die Halbkugelquerschnittsfläche 29 wird weiterhin durch die Reflexionsschicht 30 bewirkt, wenn für die Reflexionsschicht 30 ein rückstreuendes Material verwendet wird.

Um den Kontakt mit dem zu bestrahlenden Gewebe zwischen Portio und Zervix-Kanal zu verbessern, kann der Verlauf der Oberfläche der Halbkugel 26 der Anatomie angepasst werden. Zu diesem Zweck ist das in Figur 6 dargestellte abgewandelte Ausführungsbeispiel des Lichtapplikators 27 mit einem kegelförmigen Ansatz 33 versehen, der auf der Halbkugelquerschnittsfläche 29 aufsetzt und sich zum distalen Ende 10 hin verjüngt.

Um die räumliche Homogenität der aus dem Lichtapplikator 27 austretenden Strahlung weiter zu verbessern, kann die von der Schlauchoberfläche 28 und der Halbkugelquerschnittsfläche 29 gebildete Lichtaustrittsfläche, wie in Figur 7 dargestellt, mit einer partiell rückstreuenden Schicht 34 versehen sein. Wenn das Rückstrahlvermögen der rückstreuenden Schicht 34 größer ist als deren Transparenz, werden die Photonen im Mittel mehrfach in das Innere des Streukörpers 1 und der Halbkugel 26 zurückgestreut, bevor sie schließlich den Lichtapplikator 27 durch die rückstreuende Schicht 34 hindurch verlassen. Auf diese Weise wird die räumliche Lichtverteilung im Inneren des Lichtapplikators 27 homogenisiert und damit auch die Verteilung des von der rückstreuenden Schicht 34 nach außen abgegebenen Lichts.

Es sei angemerkt, dass eine der rückstreuenden Schicht 34 entsprechende Schicht auch auf das in Figur 6 dargestellte Ausführungsbeispiel des Lichtapplikators 27 aufgebracht werden kann.

Als Material für die Streumedien in den Streubereichen 7, 8 und 9 eignet sich ein hochtransparenter Silikonkautschuk, der mit TiO₂ oder BaSO₄ dotiert werden kann. Eine verarbeitungsfertige Mischung aus 50 % Farbpigmenten auf der Basis von TiO₂ und 50 % Trägermaterial auf der Basis von Silikonkautschuk ist das Material RTV-ME 601 mit der Paste FL Weiß der Firma Wacker, Burghausen. Dieses Material kann mit klarem Silikonkautschuk weiter verdünnt werden, bis die gewünschten Konzentrationen erreicht werden. Die Konzentrationen der Paste FL Weiß für den Streukörper 1 mit einer Länge von 5 cm liegen in der Größenordnung von 0,005 % bis 0,2 %. Bei höheren Konzentrationen der Streuzentren kann dieses Material auch für die rückstreuende Schicht 34 oder die Reflexionsschicht 30 verwendet werden. Darüber hinaus kann dieses Material in flüssigem Zustand verarbeitet werden und härtet bei Raumtemperatur nach einer typischen Aushärtezeit von 90 Minuten aus.

Als Material für das Schlauchstück 5 sollte ein Material verwendet werden, dessen Brechungsindex kleiner als der Brechungsindex des für die Streubereiche 7 bis 9 verwendeten Streumediums ist. In diesem Fall wird vor allem das ungestreute Licht an der Grenzfläche zwischen dem Schlauchstück 5 und den Streubereichen 7 bis 9 totalreflektiert bis es nach einem Streuvorgang unter einem Winkel auf die Grenzfläche trifft, der ein Austreten des gestreuten Lichts gestattet. Auf diese Weise wird verhindert, dass ungestreutes Licht den Streukörper 1 verlässt.

Wenn der Streukörper 1 nicht biegsam sein soll, kann anstelle des Schlauchstücks 5 ein Rohr aus Plexiglas verwendet werden. Für den Spiegel 14 am distalen Ende 10 des Streukörpers 1 kommt zum Beispiel ein Silberzylinder mit polierter Endfläche oder aber auch ein kurzes Glasfaserstück in Betracht, dessen proximale Endfläche mit Silber bedampft wurde. Zum Ausbilden der Spiegelfläche kommen auch andere auf die Wellenlänge des verwendeten Lichts abgestimmte Materialien, zum Beispiel Aluminium, in Betracht.

Die wesentlichen Vorteile der hier beschriebenen Lichtapplikatoren liegen im geringen Aufwand bei der Fertigung des Streukörpers 1 und in der großen Freiheit bei der Gestaltung der Verteilung des von den Lichtapplikatoren abgegebenen Lichts. Insbesondere ist es möglich, mit Hilfe des Streukörpers 1 eine homogene Lichtverteilung entlang des Streukörpers 1 zu erzielen. Durch die Kombination des Streukörpers 1 mit der Halbkugel 26 ergibt sich der Lichtapplikator 27, der die photodynamische Therapie von Portio und Zervix-Kanal wesentlich vereinfacht. Die Bestrahlung von Portio und Zervix-Kanal kann nunmehr in einem Durchgang ohne aufwändige Positionierung und Dosimetrieberechnungen in unmittelbarem Gewebekontakt erfolgen.

Die hier beschriebenen Lichtapplikatoren lassen sich, wie bereits erwähnt, für die photodynamische Therapie (PDT) verwenden. Daneben kommt auch eine Verwendung im Rahmen der photodynamische Diagnose (PDD) und der laserinduzierten Thermotherapie (LITT) in Betracht.

Schließlich sei noch angemerkt, dass die paraboloidförmigen Grenzflächen auch durch kegelförmige und kegelstumpfförmige, hyperboloidförmige oder dem Verlauf einer Exponentialfunktion folgende Grenzflächen ersetzt werden können. Die Grenzflächen müssen nicht notwendig rotationssymmetrisch bezüglich der Längsachse des Streukörpers ausgebildet sein. Die einzelnen Streubereiche sollten sich jedoch in einer im rechten Winkel zur Längsachse des Streukörpers oder Diffusors stehenden Sichtlinie überlappen, um einen graduellen Übergang vom einen Streubereich in den benachbarten Streubereich zu ermöglichen.

## Patentansprüche

1. Lichtapplikator mit einem an einen Lichtleiter (2) anbringbaren Streukörper (1), bei dem verschiedene Streubereiche (7, 8, 9) mit unterschiedlichen Streuparametern entlang der in den Streukörper (1) hinein verlängerten optischen Achse (6) des Lichtleiters (2) aufeinander folgen, wobei die Streubereiche (7, 8, 9) bezüglich einer im rechten Winkel zur optischen Achse (6) des Lichtleiters (2) ausgerichteten Sichtlinie überlappen,
**dadurch gekennzeichnet, dass** Grenzflächen (11, 12) zwischen Streubereichen (7, 8, 9) paraboloidförmig ausgebildet sind.

2. Lichtapplikator nach Anspruch 1,
bei dem Grenzflächen (11, 12) zwischen benachbarten Streubereichen (7, 8, 9) kegelförmig ausgebildet sind.

3. Lichtapplikator nach Anspruch 1 oder 2,
dessen Streukörper (1) an seinem distalen Ende (10) ein Spiegelelement (14) aufweist.

4. Lichtapplikator nach einem der Ansprüche 1 bis 3,
bei dem aufgrund der gewählten Streuparameter in den Streubereichen (7, 8, 9) die.Streuwahrscheinlichkeit zum distalen Ende (10) hin zunimmt.

5. Lichtapplikator nach Anspruch 4,
bei dem die über die Querschnittsfläche gemittelte Konzentration von Streuzentren entlang der optischen Achse (6) zum distalen Ende (10) des Streuköpers (1) hin ansteigt.

6. Lichtapplikator nach einem der Ansprüche 1 bis 5,
bei dem dem Streukörper (1) ein Reflexionselement (26, 30) zugeordnet ist, durch das vom Streukörper (1) emittiertes Licht in eine vorbestimmte Richtung lenkbar ist.

7. Lichtapplikator nach Anspruch 6,
bei dem das Reflexionselement ein auf den Streukörper (1) aufgebrachtes Kugelsegment (26) ist, das auf einer Außenseite mit einer Licht zurückwerfenden Schicht (30) versehen ist.

8. Lichtapplikator nach Anspruch 7,
bei dem der Übergang (33) zwischen der Licht emittierenden Oberfläche (29) des Reflexionselements (26, 30) und der Licht emittierenden Oberfläche (28) des Streukörpers (1) als kegelförmiger Ansatz (33) ausgestaltet ist.

9. Lichtapplikator nach einem der Ansprüche 6 bis 8,
bei dem die über den Querschnitt gemittelte Konzentration der Streuzentren im Bereich des Reflexionselements (26, 30) ein lokales Maximum aufweist.

10. Lichtapplikator nach Anspruch 9,
bei dem die über die Querschnittsfläche gemittelte Konzentration von Streuzentren entlang der optischen Achse (6) zwischen dem proximalen Ende (4) und dem distalen Ende (10) des Streuköpers (1) ein Minimum zeigt.

11. Lichtapplikator nach einem der Ansprüche 1 bis 10,
bei dem die Streubereiche (7, 8, 9) auf der Basis von Silikon hergestellt sind.

12. Lichtapplikator nach einem der Ansprüche 1 bis 11,
bei dem in den Streubereichen (7, 8, 9) vorhandene Streuzentren auf der Basis von TiO₂ oder BaSO₄ hergestellt sind.

13. Lichtapplikator nach einem der Ansprüche 1 bis 12,
bei dem die Streubereiche (7, 8, 9) von einer Hülle umgeben sind, die einen kleineren Brechungsindex als der Brechungsindex der Streubereiche (7, 8, 9) aufweist.

14. Lichtapplikator nach einem der Ansprüche 1 bis 13,
dessen Licht emittierende Oberflächen (28, 29) von einer partiell zurückstreuenden Schicht (34) überzogen sind.

15. Lichtapplikator nach einem der Ansprüche 1 bis 14,
dessen Streukörper (1) biegsam ausgebildet ist.

16. Lichtapplikator nach einem der Ansprüche 1 bis 14,
dessen Streukörper (1) starr ausgebildet ist.

17. Verfahren zur Herstellung eines an einen Lichtleiter (2) anschließbaren Streukörpers (1), bei dem verschiedene Streubereiche (7, 8, 9) mit unterschiedlichen Streuparametern entlang der in den Streukörper (1) hinein verlängerten Achse (6) des Lichtleiters (2) ausgebildet werden, wobei
- für den Streukörper (1) ein Hohlkörper (5) verwendet wird, der abschnittsweise mit einem ersten Streumedium (15) gefüllt wird, und
- in das erste Streumedium (15) ein zweites Streumedium (19) injiziert wird,
**dadurch gekennzeichnet, dass** bei dem aufgrund laminarer Strömung des zweiten Streumediums (19) im ersten Streumedium (15) eine paraboloidförmige Grenzfläche (11) zwischen dem ersten Streumedium (15) und dem zweiten Streumedium (19) ausgebildet wird.

18. Verfahren nach Anspruch 17,
bei dem das erste Streumedium (15) und das zweite Streumedium (19) jeweils in den Hohlkörper (5) eingesaugt werden.

19. Verfahren nach Anspruch 17 oder 18,
bei dem das zweite Streumedium (19) von einem ersten Ende (10) des Hohlkörpers (5) in das erste Streumedium (15) injiziert wird und ein drittes Streumedium (24) von einem zweiten Ende (4) des Hohlkörpers (5) in das erste Streumedium (15) injiziert wird.

20. Verfahren nach einem der Ansprüche 17 bis 19,
bei dem die Streumedien (15, 19 24) ausgehärtet werden.

## Claims

1. A light applicator with a diffusor (1) which can be attached to a light guide (2) and in which different diffusion regions (7, 8, 9) with different scattering parameters follow successively along the optical axis (6) of the light guide (2) extending into the diffuser (1), wherein the diffusion regions (7, 8, 9) will overlap with respect to a visual line aligned at a right angle to the optical axis (6) of the light guide (2),
**characterized in that**
boundary surfaces (11, 12) are formed in a paraboloidal way between diffusion regions (7, 8, 9).

2. The light applicator according to claim 1,
wherein the boundary surfaces (11, 12) are formed in a conical way between adjacent diffusion regions (7, 8, 9).

3. The light applicator according to claim 1 or 2,
whose diffusor (1) comprises a mirror element (14) at its distal end (10).

4. The light applicator according to any one of the claims 1 to 3,
wherein the scattering probability increases towards the distal end (10) due to the chosen scattering parameters in the diffusion regions (7, 8, 9).

5. The light applicator according to claim 4,
wherein the concentration of scattering centers as averaged over the cross-sectional surface area increases along the optical axis (6) towards the distal end (10) of the diffusor (1).

6. The light applicator according to any one of the claims 1 to 5,
wherein the diffusor (1) is associated with a reflection element (26, 30) by which the light emitted by the diffusor (s) can be guided in a predetermined direction.

7. The light applicator according to claim 6,
wherein the reflection element is a spherical segment (26) which is applied on the diffusor (1) and which is provided on one outer side with a layer (30) reflecting the light.

8. The light applicator according to claim 7,
wherein the transition (33) between the light-emitting surface (29) of the reflection element (26, 30) and the light-emitting surface (28) of the diffusor (1) has the shape of a conical nose.

9. The light applicator according to any one of claims 6 to 8,
wherein the concentration of the scattering centers as averaged over the cross section has a local maximum in the region of the reflection element (26, 30).

10. The light applicator according to claim 9,
wherein the concentration of scattering centers along the optical axis (6) as averaged over the cross-sectional surface area shows a minimum between the proximal end (4) and the distal end (10) of the diffusor (1).

11. The light applicator according to any one of the claims 1 to 10,
wherein the diffusion regions (7, 8, 9) are produced on the basis of silicone.

12. The light applicator according to any one of the claims 1 to 11,
wherein scattering centers present in the diffusion regions (7, 8, 9) are produced on the basis of TiO₂ or BaSO₄.

13. The light applicator according to any one of the claims 1 to 12,
wherein the diffusion regions (7, 8, 9) are enclosed by a covering which has a smaller refractive index than the refractive index of the diffusion regions (7, 8, 9).

14. The light applicator according to any one of the claims 1 to 13,
whose light-emitting surfaces (28, 29) are covered by a partly backscattering layer (34).

15. The light applicator according to any one of the claims 1 to 14,
whose diffusor (1) is provided with a flexible configuration.

16. The light applicator according to any one of the claims 1 to 14,
whose diffusor (1) is provided with a rigid configuration.

17. A method for producing a diffusor (1) which can be connected to a light guide (2) and in which different diffusion regions (7, 8, 9) with different scattering parameters are formed along the axis (6) of the light guide extending into the diffusor (1), wherein
- a hollow body (5) is used for the diffusor (1) which is filled in sections with a first diffusion medium (15), and wherein
- a second diffusion medium (19) is injected into the first diffusion medium (15),
**characterized in that**
in the first diffusion medium (15) a paraboloidal boundary surface (11) is formed between the first diffusion medium (15) and the second diffusion medium (19) as a result of the laminar flow of the second diffusion medium (19) in the first diffusion medium (15).

18. The method according to claim 17,
wherein the first diffusion medium (15) and the second diffusion medium (19) are each sucked into the hollow body (5).

19. The method according to claims 17 or 18,
wherein the second diffusion medium (19) is injected from a first end (10) of the hollow body (5) into the first diffusion medium (15) and a third diffusion medium (24) is injected from a second end (4) of the hollow body (5) into the first diffusion medium (15).

20. The method according to any one of claims 17 to 19, wherein the diffusion media (15, 19, 24) are cured.

## Revendications

1. Applicateur de lumière avec un diffuseur (1) pouvant être monté sur un conducteur optique (2), pour lequel différentes zones de diffusion (7, 8, 9) avec différents paramètres de diffusion se suivent le long de l'axe optique (6) prolongé dans le diffuseur (1) du conducteur optique (2), les zones de diffusion (7, 8, 9) se chevauchant par rapport à une ligne visuelle orientée à angle droit par rapport à l'axe optique (6) du conducteur optique (2),
**caractérisé en ce que** des surfaces limites (11, 12) entre des zones de diffusion (7, 8, 9) sont réalisées en forme de parabole.

2. Applicateur de lumière selon la revendication 1, pour lequel des surfaces limites (11, 12) entre des zones de diffusion contigües (7, 8, 9) sont réalisées en forme de cône.

3. Applicateur de lumière selon la revendication 1 ou 2, dont le diffuseur (1) présente sur son extrémité distale (10) un élément de miroir (14).

4. Applicateur de lumière selon l'une quelconque des revendications 1 à 3, pour lequel en raison des paramètres de diffusion choisis dans les zones de diffusion (7, 8, 9), la probabilité de diffusion augmente vers l'extrémité distale (10).

5. Applicateur de lumière selon la revendication 4, pour lequel la concentration moyenne sur l'aire de section des centres de diffusion augmente le long de l'axe optique (6) vers l'extrémité distale (10) du diffuseur (1).

6. Applicateur de lumière selon l'une quelconque des revendications 1 à 5, pour lequel au diffuseur (1) est associé un élément de réflexion (26, 30), par lequel de la lumière émise par le diffuseur (1) peut être dirigée dans une direction prédéterminée.

7. Applicateur de lumière selon la revendication 6, pour lequel l'élément de réflexion est un segment sphérique (26) appliqué sur le diffuseur (1) qui est pourvu sur un côté extérieur d'une couche (30) reflétant la lumière.

8. Applicateur de lumière selon la revendication 7, pour lequel le passage (33) entre la surface émettant la lumière (29) de l'élément de réflexion (26, 30) et la surface (28) émettant la lumière du diffuseur (1) est configuré comme une saillie sphérique.

9. Applicateur de lumière selon l'une quelconque des revendications 6 à 8, pour lequel la concentration moyenne sur la section de centres de diffusion présente dans la zone de l'élément de réflexion (26, 30) un maximum local.

10. Applicateur de lumière selon la revendication 9, pour lequel la concentration moyenne sur l'aire de section de centres de diffusion présente le long de l'axe optique (6) entre l'extrémité proximale (4) et l'extrémité distale (10) du diffuseur (1), un minimum.

11. Applicateur de lumière selon l'une quelconque des revendications 1 à 10, pour lequel les zones de diffusion (7, 8, 9) sont fabriquées à base de silicone.

12. Applicateur de lumière selon l'une quelconque des revendications 1 à 11, pour lequel des centres de diffusion présents dans les zones de diffusion (7, 8, 9) sont fabriqués à base de TiO₂ ou de BaSO₄.

13. Applicateur de lumière selon l'une quelconque des revendications 1 à 12, pour lequel les zones de diffusion (7, 8, 9) sont entourées d'une enveloppe qui présente un indice de réfraction plus petit que l'indice de réfraction des zones de diffusion (7, 8, 9).

14. Applicateur de lumière selon l'une quelconque des revendications 1 à 13, dont les surfaces (28, 29) émettant de la lumière sont recouvertes d'une couche (34) à rediffusion partielle.

15. Applicateur de lumière selon l'une quelconque des revendications 1 à 14, dont le diffuseur (1) est réalisé de manière flexible.

16. Applicateur de lumière selon l'une quelconque des revendications 1 à 14, dont le diffuseur (1) est réalisé de manière rigide.

17. Procédé de fabrication d'un diffuseur (1) pouvant être raccordé à un conducteur optique (2), pour lequel différentes zones de diffusion (7, 8, 9) avec différents paramètres de diffusion sont réalisées le long de l'axe (6) prolongé dans le diffuseur (1) du conducteur optique (2),
- pour le diffuseur (1) étant utilisé un corps creux (5) qui est rempli par endroits d'un premier moyen de diffusion (15), et
- dans le premier moyen de diffusion (15) étant injecté un second moyen de diffusion (19),
**caractérisé en ce que** pour celui-ci, en raison de l'écoulement laminaire du second moyen de diffusion (19) dans le premier moyen de diffusion (15), une surface limite (11) en forme de parabole est réalisée entre le premier moyen de diffusion (15) et le second moyen de diffusion (19).

18. Procédé selon la revendication 17, pour lequel le premier moyen de diffusion (15) et le second moyen de diffusion (19) sont absorbés respectivement dans le corps creux (5).

19. Procédé selon la revendication 17 ou 18, pour lequel le second moyen de diffusion (19) est injecté par une première extrémité (10) du corps creux (5) dans le premier moyen de diffusion (15) et un troisième moyen de diffusion (24) est injecté par une seconde extrémité (4) du corps creux (5) dans le premier moyen de diffusion (15).

20. Procédé selon l'une quelconque des revendications 17 à 19, pour lequel les moyens de diffusion (15, 19, 24) sont durcis.
